# EUROPEAN PATENT SPECIFICATION

(11) **EP 3 357 526 B1**
(45) Date of publication and mention of the grant of the patent: **26.06.2019**
(21) Application number: 17154234.3
(22) Date of filing: 01.02.2017
(51) Int. Cl.: A61M 5/14, A61M 5/162, A61M 39/12, A61M 39/10

(54) **SPIKE CONNECTOR**
EINSTECHDORN
CONNECTEUR À AIGUILLE

(43) Date of publication of application: 08.08.2018
(73) Proprietor: Micrel Medical Devices S.A., 15344 Gerakas (GR)
(72) Inventor: TSOUKALIS, Achilleas, 19013 ANAVYSSOS (ATTIKI) (GR)
(74) Representative: Eisenführ Speiser

(56) References cited:
- EP-A1- 0 937 480
- WO-A1-98/56448
- GB-A- 2 171 678
- US-A1- 2005 209 581

## Description

The present invention relates to a spike connector, comprising a spike, a gripping body carrying the spike and having side walls which define at least one external gripping surface adapted to enable a user to grip the gripping body for coupling the spike with a reservoir, and a tube connector provided at the gripping body and fluidly communicating with the spike wherein the tube connector is adapted to be connected to a tube.

In the prior art, for a gravity or pump infusion, a fluid container or reservoir is connected to an infusion line through a spike connector of the aforementioned kind. The spike of the spike connector is plugged into a tube-shaped socket at the bottom of the fluid reservoir and retained therein by means of friction forces. Usually, the dimensions of the spike are standardized.

As shown in figure 1 the prior art spike connector which is described e.g. in EP 1 277 490 A1 is built up by three sections A, B and C.

Section A comprises a spike 2 which is usually a dual lumen spike having standardized outside dimensions.

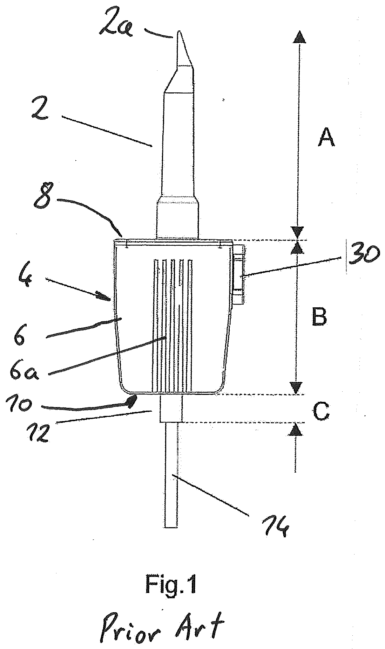

Section B comprises a gripping body 4 having side walls 6 and a spike plate 8 which carries the spike 2 and closes the gripping body 4 towards the spike 2. The bottom 10 at the opposite end relative to the spike plate 8 is also closed by a plate (not shown in figure 1). The side wall 6 shown in figure 1 defines an outside or external gripping surface 6a which is adapted to enable a user to grip the gripping body 4 for coupling the spike 2 with a fluid reservoir (not shown).

Section C comprises a tube connector 12 to which a tube 14 is coupled as shown in figure 1. As further shown in figure 1, the connector 12 is arranged at the bottom 10 of the gripping body 4 so that the connector 12 protrudes from the bottom 10 of the gripping body 4.

So, the three sections A, B and C are provided in-line, e.g. spike, handling and then connection functionality, wherein the tube connector 12 is located at the bottom 10 of the gripping body 4 so as to form a protrusion. Such an arrangement of the tube connector 12 does not cause any problems if the fluid reservoir is hanged on a pole. However, the risk of upstream occlusion in the tube 14 dramatically increases in particular if the tube 14 is subject to forces at the tube connector 12 where the tube 14 bends sharply at a very small bending radius so as to form a kinking point. This might be, for instance, the case if the fluid reservoir and the spike connector coupled thereto are put in a rucksack as usually in parenteral nutrition e.g. by accidentally sitting at the bottom of the rucksack, causing nuisance to the user due to upstream occlusion alarm on the infusion pump. Document GB 2171678 discloses features of the preamble of claim 1.

It is an object of the present invention to provide an improved spike connector wherein the risk for kinking the coupled tube is reduced or even removed.

In order to achieve the above in further objects, according to the present invention, there is provided a spike connector, comprising a spike, a gripping body carrying the spike and having side walls which define at least one external gripping surface adapted to enable a user to grip the gripping body for coupling the spike with a reservoir, and a tube connector provided at the gripping body and fluidly communicating with the spike wherein the tube connector is adapted to be connected to a tube, wherein the tube connector is arranged within the gripping body, wherein at least one portion of the side walls of the gripping body extends beyond the tube connector at a region of the gripping body where the tube is to be connected to the tube connector.

According to the teaching of the present invention, in the region of the bottom the gripping body is provided with somewhat like an open recess or cavity accommodating an external tube connected to the tube connector whose free end is positioned above the open bottom of the gripping body. So, the free end of the tube connector precedes the open bottom of the gripping body and, hence, lies within the gripping body at a level spaced from the level defined by the open bottom of the gripping body. In other words, the free end of the tube connector lies within the gripping body above or higher than the open bottom of the gripping body wherein "higher" means an orientation from the bottom towards the interior of the gripping body. This is achieved by that the tube connector is arranged within the gripping body, wherein at least one portion of the side walls of the gripping body extends beyond the tube connector at a location or region of the gripping body where an external tube is to be connected to the tube connector. So, the tube connected to the tube connector is given enough space to bend at a larger bending radius until reaching and exiting the bottom end of the gripping body without any kinking. Due to the aforementioned arrangement according to the present invention, the risk to bend a tube connected to the tube connector so that a kinking and, hence, occlusion occurs is dramatically reduced and even removed.

Preferred embodiments and modifications of the present invention are defined in the dependent claims.

According to a preferred embodiment, the tube connector is at least partly surrounded by the side walls of the gripping body which, hence, restrict the open recess or cavity accommodating the tube connector.

Further, preferably the side walls of the gripping body comprise end portions which at least partly define protrusions relative to the tube connector so as to space the bottom of the gripping body from the tube connector.

According to a further preferred embodiment, the side walls of the gripping body comprise at least one end portion extending beyond the tube connector and having a free end which is provided with an open recess. Such an open recess gives enough space for the bent tube to leave the gripping body and, hence, the spike connector without being clamped so as to avoid the risk of a further occlusion.

Preferably, the highest point of the open recess does not lie higher than the free end of the tube connector and most preferably between the level of the free end of the tube connector and the level of the bottom of the gripping body. According to a preferred modification, the open recess has a bent or rounded shape in particular like an arc; due to this modification, it is ensured that the tube is caused to slide by itself to the highest point of the open recess so that it is not occluded by the side wall.

According to a further preferred embodiment, the side walls comprise at least one end portion extending beyond the tube connector and having a free end which is provided with at least one protrusion. According to a preferred modification of this embodiment, the protrusion has a rounded tip or peak; the provision of such a rounded protrusion is advantageous in that in case the tube accidentally lies under the side wall in the region of the protrusion the rounded shape of its tip or peak causes the tube to slide by itself along a side edge of the protrusion away from its tip or peak so as not to be occluded.

In a further modification of the aforementioned embodiment, the free end of the at least one end portion of the side walls is provided with two protrusions spaced from each other. In this modification, the open recess is preferably provided between these two protrusions so that the tube when sliding along the edge of a protrusion is automatically guided into the open recess and in particular to its highest point.

According to a further preferred embodiment, the at least one end portion of the side walls is configured as a plate.

In the following a preferred embodiment of the present invention is described with reference to the enclosed figures, wherein
- Fig. 1: shows a prior art spike connector;
- Fig. 2: shows a spike connector according to a preferred embodiment with the tube connected to the tube connector being bent towards a broad side wall in a perspective view (a), in a side view (b) and in a longitudinal section view (c); and
- Fig. 3: shows a spike connector of fig. 2 with the tube connected to the tube connector being bent towards a narrow side wall in a perspective view (a), in a side view (b) and in a longitudinal section view (c).

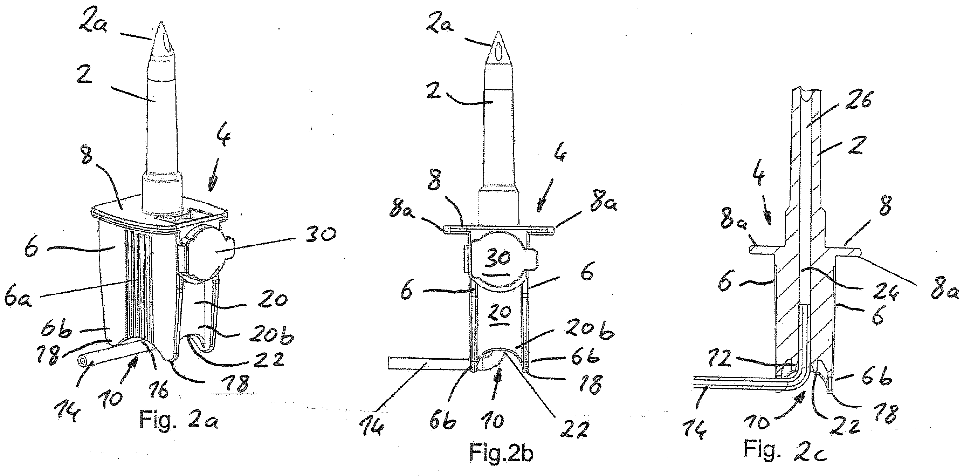

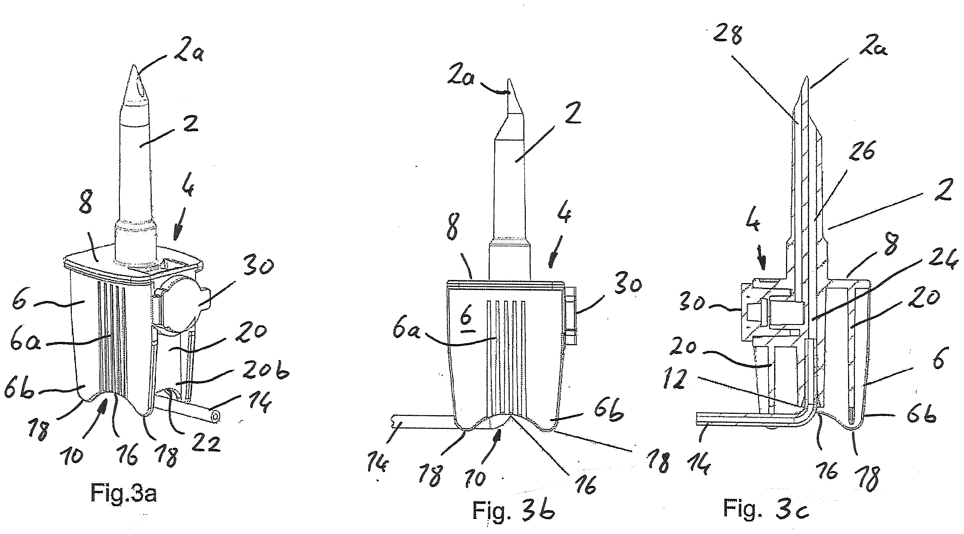

In the figures 2 and 3 it is shown a spike connector according to a preferred embodiment of the present invention which mainly differs from the prior art spike connector shown in fig. 1 and described above with reference to fig. 1 in that the bottom 10 of the gripping body 4 is open or at least provided with an open recess or cavity and the tube connector 12 is arranged within the gripping body 4 inwards from the bottom 10 and, hence, spaced from the bottom 10. A further difference from the prior art spike connector of fig. 1 is that the end portion 6b of the side walls 6 in the region of the bottom 10 is provided with an open recess 16 having a bent shape like an arc. Further, according to the preferred embodiment shown, the end portion 6b of the side walls 6 in the region of the bottom 10 of the gripping body 4 comprises two protrusions 18 having a rounded peak or tip wherein both these rounded protrusions 18 are spaced from each other with the open recess 16 being arranged therebetween.

As further seen from the figures 2 and 3, the gripping body 4 of the spike connector according to the preferred embodiment comprises two side walls 6 spaced from each other and two further side walls 20 spaced from each other and oriented transversely to the side walls 6. In the preferred embodiment shown, the side walls 6 defining the external gripping surface 6a are wider than the further side walls 20 so that the side walls 6 are broad side walls and the further side walls 20 are narrow side walls. Similar to the open recess 16 of the end portion 6b of the broad side walls 6, the end portion 20b of the narrow side walls 20 in the region of the bottom 10 of the gripping body 4 are also provided with an open recess 22 having a bent shape like an arc.

The open recess 16 of the end portion 6b of the broad side walls 6 and the open recess 22 of the end portion 20b of the narrow side walls 20 each give enough space for the bent tube 14 to leave the gripping body 4 and, hence, the whole spike connector without being clamped so as to avoid the risk of a further occlusion. In fig. 2 the tube 14 is shown leaving the gripping body 4 through the open recess 16 of the end portion 6b of one of the broad side walls 6, whereas in fig. 3 the tube 14 is shown leaving the gripping body 4 through the open recess 22 of the end portion 20b of one of the narrow side walls 20. As shown in figures 2c and 3c, the highest point of the open recesses 16 and 22 lies shortly below the level of the free end of the tube connector 12. Since the open recesses 16, 22 have a bent shape like an arc, it is ensured that the tube 14 is caused to slide by itself to the highest point of the open recess 16 or 22 so that it is not occluded by the respective side wall 6 or 20 as shown in figures 2a and 3a. Moreover, since the two protrusions 18 of the end portion 6b of the broad side walls 6 have a rounded peak or tip, the tube 14 is caused to slide by itself from the rounded tip or peak of a protrusion 16 into the open recess 16 of the end portion 6b of the respective broad side wall 6 or possibly into the open recess 22 of the end portion 20b of the respective narrow side wall 20 and in particular to the highest point of the open recess 16 or 22 so as not to be occluded as well.

The side walls 6 and 20 of the gripping body 4 consist of plates whose end portions defining the end portions 6b, 20b of the side walls 6, 20 in the region of the bottom 10 extend beyond the tube connector 8.

As shown in the figures 2a, 3a and 3b, the gripping surface 6a of both the broad side walls 6 are provided with a plurality of narrow ribs extending in the same orientation as the spike 2.

As shown in figures 2c and 3c, the tube connector 8 fluidly communicates with a passage 24 which again fluidly communicates with a first passage way 26 of two passage ways 26, 28 provided within the spike 2. The first passage way 26 within the spike 2 defines a first lumen for fluid connection and the second passage way 26 within the spike 2 defines a second lumen for air ventilation of the fluid reservoir (not shown) so that the shown spike 2 is embodied as a dual lumen spike. The second passage way 28 of the spike 2 is a part of a conventional vent path as already used in the prior art and communicates with a an air vent assembly 30 which is provided at one of the narrow side walls 20 and includes a hydrophobic membrane (not shown) and an air-tight cap (not designated in the figures) to be opened or closed in accordance with infusion needs as usual.

As in particular shown in the figures 2b and 2c, the spike plate 8 is provided with extensions 8a which define somewhat like a limitation, barrier or stop with regard to the gripping surface 6a in order to avoid that the spike 2 is touched by a user's hand.

The spike 2 comprises a sharp tip 2a as known from conventional spikes for penetrating rubber taps of medication reservoirs or bottles or of vials (all not shown here). Further, the spike connector and in particular its gripping body 4 can be of clear colour for the purpose of UV bonding the tube 14 to the tube connector 12 or of any colour for solvent bonding and of biocompatible material depending on the fluids to be infused.

## Claims

1. A spike connector, comprising
a spike (2),
a gripping body (4) carrying the spike (2) and having side walls (6; 20) which define at least one external gripping surface (6a) adapted to enable a user to grip the gripping body (4) for coupling the spike (2) with a reservoir, and
a tube connector (12) provided at the gripping body (4) and fluidly communicating with the spike (2) wherein the tube connector (12) is adapted to be connected to a tube (14),
**characterized in that** the tube connector (12) is arranged within the gripping body (4), wherein at least one portion of the side walls (6; 20) of the gripping body (4) extends beyond the tube connector (12) at a region of the gripping body (4) where the tube (14) is to be connected to the tube connector (12).

2. The spike connector according to claim 1, wherein the tube connector (12) is at least partly surrounded by the side walls (6; 20) of the gripping body (4).

3. The spike connector according to claim 1 or 2, wherein the side walls (6; 20) of the gripping body (4) comprise end portions (6b; 20b) which at least partly define protrusions relative to the tube connector (12).

4. The spike connector according to at least anyone of the preceding claims, wherein the side walls (6; 20) of the gripping body (4) comprise at least one end portion (6b; 20b) extending beyond the tube connector (12) and having a free end which is provided with an open recess (16; 22).

5. The spike connector according to claim 4, wherein the open recess (16; 22) has a bent or rounded shape.

6. The spike connector according to at least anyone of the preceding claims, wherein the side walls (6; 20) comprise at least one end portion (6b; 20b) extending beyond the tube connector (12) and having a free end which is provided with at least one protrusion (18).

7. The spike connector according to claim 6, wherein the protrusion (18) has a rounded tip or peak.

8. The spike connector according to claim 6 or 7, wherein the free end of the at least one end portion (6b) of the side walls (6) is provided with two protrusions (18) spaced from each other.

9. The spike connector according to claims 4 or 5 as well as at least anyone of the claims 6 to 8, wherein the open recess (16) is provided between the two protrusions (18).

10. The spike connector according to at least anyone of the preceding claims, wherein the at least one end portion (6b; 20b) of the side walls (6; 20) is configured as a plate.

## Patentansprüche

1. Einstechdorn, mit
einem Dorn (2),
einem den Dorn (2) tragenden und Seitenwände (6; 20) aufweisenden Greifkörper (4), der mindestens eine externe Greiffläche (6a) aufweist, die ausgebildet ist, einem Benutzer das Greifen des Greifkörpers (4) zur Verbindung des Dorns (2) mit einem Reservoir zu ermöglichen, und
einem Schlauchverbinder (12), der am Greifkörper (4) vorgesehen ist und sich in fluider Verbindung mit dem Dorn (2) befindet, wobei der Schlauchverbinder (12) ausgebildet ist, mit einem Schlauch (14) verbunden zu werden,
**dadurch gekennzeichnet, dass** der Schlauchverbinder (12) innerhalb des Greifkörpers (4) angeordnet ist, wobei sich mindestens ein Abschnitt der Seitenwände (6; 20) des Greifkörpers (4) über den Schlauchverbinder (12) in einem Bereich des Greifkörpers (4) hinaus erstreckt, wo der Schlauch (14) mit dem Schlauchverbinder (12) zu verbinden ist.

2. Einstechdorn nach Anspruch 1, bei welchem der Schlauchverbinder (12) zumindest teilweise von den Seitenwänden (6; 20) des Greifkörpers (4) umgeben ist.

3. Einstechdorn nach Anspruch 1 oder 2, bei welchem die Seitenwände (6; 20) des Greifkörpers (4) Endabschnitte (6b; 20b) aufweisen, die zumindest teilweise Vorsprünge gegenüber dem Schlauchverbinder (12) bilden.

4. Einstechdorn nach mindestens einem der vorangegangenen Ansprüche, bei welchem die Seitenwände (6; 20) des Greifkörpers (4) mindestens einen Endabschnitt (6b; 20b) aufweisen, der sich über den Schlauchverbinder (12) hinaus erstreckt und ein freies Ende besitzt, das mit einer offenen Aussparung (16; 22) versehen ist.

5. Einstechdorn nach Anspruch 4, bei welchem die offene Aussparung (16; 22) eine gekrümmte oder gerundete Form besitzt.

6. Einstechdorn nach mindestens einem der vorangegangenen Ansprüche, bei welchem die Seitenwände (6; 20) mindestens einen Endabschnitt (6b; 20b) aufweisen, der sich über den Schlauchverbinder (12) hinaus erstreckt und ein freies Ende besitzt, das mit mindestens einem Vorsprung (18) versehen ist.

7. Einstechdorn nach Anspruch 6, bei welchem der Vorsprung (18) eine abgerundete Spitze besitzt.

8. Einstechdorn nach Anspruch 6 oder 7, bei welchem das freie Ende des mindestens einen Endabschnittes (6b) der Seitenwände (6) mit zwei voneinander beabstandeten Vorsprüngen (18) versehen ist.

9. Einstechdorn nach Anspruch 4 oder 5 sowie nach mindestens einem der Ansprüche 6 bis 8, bei welchem die offene Aussparung (16) zwischen zwei Vorsprüngen (18) vorgesehen ist.

10. Einstechdorn nach mindestens einem der vorangegangenen Ansprüche, bei welchem der mindestens eine Endabschnitt (6b; 20b) der Seitenwände (6;20) als Platte ausgebildet ist.

## Revendications

1. Connecteur à aiguille, comprenant
une aiguille (2),
un corps de préhension (4) portant l'aiguille (4) et ayant des parois latérales (6 ; 20) qui définissent au moins une surface de préhension externe (6a) adaptée pour permettre à un utilisateur de saisir le corps de préhension (4) pour accoupler l'aiguille (2) avec un réservoir, et
un connecteur de tube (12) situé au niveau du corps de préhension (4) et communiquant fluidiquement avec l'aiguille (2) dans lequel le connecteur de tube (12) est adapté pour être connecté à un tube (14),
**caractérisé en ce que** le connecteur de tube (12) est agencé dans le corps de préhension (4), dans lequel au moins une portion des parois latérales (6 ; 20) du corps de préhension (4) s'étend au-delà du connecteur de tube (12) au niveau d'une région du corps de préhension (4) où le tube (14) doit être connecté au connecteur de tube (12).

2. Connecteur à aiguille selon la revendication 1, dans lequel le connecteur de tube (12) est au moins partiellement entouré par les parois latérales (6 ; 20) du corps de préhension (4).

3. Connecteur à aiguille selon la revendication 1 ou 2, dans lequel les parois latérales (6 ; 20) du corps de préhension (4) comprennent des portions d'extrémité (6b ; 20b) qui définissent au moins partiellement des saillies par rapport au connecteur de tube (12).

4. Connecteur à aiguille selon au moins une quelconque des revendications précédentes, dans lequel les parois latérales (6 ; 20) du corps de préhension (4) comprennent au moins une portion d'extrémité (6b ; 20b) s'étendant au-delà du connecteur de tube (12) et ayant une extrémité libre qui est pourvue d'un évidement ouvert (16 ; 22).

5. Connecteur à aiguille selon la revendication 4, dans lequel l'évidement ouvert (16 ; 22) a une forme pliée ou arrondie.

6. Connecteur à aiguille selon au moins une quelconque des revendications précédentes, dans lequel les parois latérales (6 ; 20) comprennent au moins une portion d'extrémité (6b ; 20b) s'étendant au-delà du connecteur de tube (12) et ayant une extrémité libre qui est pourvue d'au moins une saillie (18).

7. Connecteur à aiguille selon la revendication 6, dans lequel la saillie (18) a un pic ou pointe arrondi.

8. Connecteur à aiguille selon la revendication 6 ou 7, dans lequel l'extrémité libre de l'au moins une portion d'extrémité (6b) des parois latérales (6) est pourvue de deux saillies (18) espacées l'une de l'autre.

9. Connecteur à aiguille selon les revendications 4 ou 5 ainsi que d'au moins une quelconque des revendications 6 à 8, dans lequel l'évidement ouvert (16) se situe entre les deux saillies (18).

10. Connecteur à aiguille selon au moins une quelconque des revendications précédentes, dans lequel l'au moins une portion d'extrémité (6b ; 20b) des parois latérales (6 ; 20) est configurée comme une plaque.
